# EUROPÄISCHE PATENTSCHRIFT

(11) **EP 1 058 117 B1**
(45) Veröffentlichungstag und Bekanntmachung des Hinweises auf die Patenterteilung: **20.06.2001**
(21) Anmeldenummer: 99250161.9
(22) Anmeldetag: 21.05.1999
(51) Int. Cl.: G01N 33/68, G01N 33/53, C07K 14/47

(54) **Verwendung von SLIM3 zur Bindung an Moleküle**
Use of SLIM3 to bind to molecules
Utilisation de SLIM3 pour la liaison aux molécules

(43) Veröffentlichungstag der Anmeldung: 06.12.2000
(73) Patentinhaber: SCHERING AKTIENGESELLSCHAFT, 13342 Berlin (DE)
(72) Erfinder: Schüle, Roland, Dr., 79367 Weisweil (DE); Müller, Judith, Dr., 79108 Freiburg (DE)

(56) Entgegenhaltungen:
- CHAN K K ET AL: "Molecular cloning and characterization of FHL2, a novel LIM domain protein preferentially expressed in human heart" GENE: AN INTERNATIONAL JOURNAL ON GENES AND GENOMES,GB,ELSEVIER SCIENCE PUBLISHERS, BARKING, Bd. 210, Nr. 2, 14. April 1998 (1998-04-14), Seiten 345-350, XP004118187 ISSN: 0378-1119
- GENINI, M. ET AL.: "Substractive Cloning and Characterization of DRAL, a Novel LIM-Domain Protein Down-Regulated in Rhabdomyosarcoma" DNA AND CELL BIOLOGY, Bd. 16, Nr. 4, April 1997 (1997-04), Seiten 433-442, XP000874394
- MORGAN, M.J. AND MADGWICK, A.J.A.: "SLIM Defines a Novel Family of LIM-Proteins Expressed in Skeletal Muscle" BIOCHEMICAL AND BIOPHYSICAL RESEARCH COMMUNICATIONS, Bd. 225, 1996, Seiten 632-638, XP000867716

## Beschreibung

Die Erfindung betrifft die in vitro Verwendung von dem Protein SLIM3 zur Bindung an Rezeptoren, weiterhin ein Verfahren zum Screening von Liganden, welche unter anderem an SLIM3 binden.

### Stand der Technik

Das Protein SLIM3 wurde von M.J. MORGAN and A.J.A. MADGWICK (1996) Biochem. Biophys, Res. Commun., Vol. 225, pp 632 - 638 zuerst beschrieben. SLIM3 besitzt 279 Aminosäuren.
SLIM3 wird stark im Herzmuskel und schwach in der Plazenta, in Skelettmuskeln, in der Prostata, in den Hoden, im Darm und in den Ovarien exprimiert. Im Gehirn, der Lunge, der Leber, den Nieren, der Pankreas, der Milz, dem Thymus und in Leukozyten konnte das Protein SLIM3 nicht nachgewiesen werden. (M. GENINI et al. (1997) DNA Cell Biol., Vol. 16, pp 433 - 442). Obgleich Expression festgestellt werden konnte, wurde die physiologische oder pathophysiologische Funktion von SLIM3 noch nicht beschrieben. Die Genfamilie der LIM - Proteine, zu der SLIM3 gehört, ist dafür bekannt, Domänen zu besitzen, welche an andere Proteine binden.

Der Kern - Rezeptor AR (Androgen - Rezeptor) und seine Funktion ist beschrieben in A.C.B. CATO et al. (1998) The androgen receptor as mediator of gene expression and signal transduction pathways, TEM, Vol. 9, pp 150 - 154 und Z.X. ZHOU et al. (1994) The androgen receptor: an overview, Recent Prog Horm Res, Vol. 49, pp 249 - 274 und E.M. WILSON et al. (1991) Molecular analysis of the androgen receptor, Ann N Y Sci, Vol. 637, pp 56 - 63.
Der Kern - Rezeptor ERβ und seine Funktion (Östrogenrezeptor β) ist beschrieben von V. GIGUERE et al. (1998) Estrogen receptor beta: re-evaluation of estrogen and antiestrogen signaling; Steriods Vol. 63, pp 335-339 und G.G. KUIPER et al. (1997) The novel estrogen receptor subtype: potential role in the cell- and promoter - specific actions of estrogens and anti-estrogens, FEBS Lett, Vol. 410: pp 87- 90.
Die zuvor genannten Kern - Rezeptoren binden an Promotoren, welche in natürlichem Umfeld antwortende (responsive) Gene, wie Probasin, MMTV (Mouse mammary tumor virus), C3 und Retinsäurerezeptor alpha 1 regulieren (M.G. PARKER et al. (1987) Identification of androgen response elements in mouse mammary tumor virus and the rat C3 gene. J. Ce.. Biochem Vol. 35, pp 285 - 292 und F. CLAESSENS et al. (1996) The androgen - specific probasin response element 2 interacts differentially with androgen and glucocorticoid receptors, J. Biol Chem, Vol. 271, pp 19013- 19016 und A. ZOU et al (1999) Estrogen receptor beta activates the human retinoic acid receptor alpha- 1 promoter in response to tamoxifen and other estrogen receptor antagonists, but not in response to estrogen, Mol Endocrinol Vol 13, pp 418 - 430) (J. SAMBROOK et al. (1989) Molecular cloning. Cold Spring Harbour Laboratory Press, New York).

### Aufgabe und Lösung

Es ist Aufgabe der Erfindung, SLIM3 gezielt mit Proteinen interagieren zu lassen, insbesondere, wenn dadurch Liganden, die die Interaktion zwischen SLIM3 und den Proteinen beeinflussen, identifiziert werden können.
Die Aufgabe wird gelöst durch die in vitro Verwendung von SLIM3 (= Protein) oder einer Modifizierung davon zur Bindung an mindestens einen der Kern - Rezeptoren
(i) AR (Androgenrezeptor) (= Protein) oder einer Modifizierung davon und
(ii) ERβ (Östrogenrezeptor β) (= Protein) oder einer Modifizierung davon,
   wobei die Modifizierung darin besteht, daß bis zu zehn Aminosäuren in dem jeweiligen modifizierten Protein gegenüber dem entsprechenden natürlichen Protein deletiert, substituiert oder inseriert sind,
   ohne dabei die Funktion des jeweiligen modifizierten Proteins im Vergleich zu dem entsprechenden natürlichen Protein zu beeinflussen.

Die Erfindung umfaßt weiterhin die in vitro Verwendung der Aminosäuresequenz für SLIM3 (= Protein),
welches von einer cDNA kodiert wird, zur Bindung an mindestens eine Aminosäuresequenz für die Kern - Rezeptoren (= Protein)
(i) AR (Androgenrezeptor), welcher von einer cDNA kodiert wird, und
(ii) ERβ (Östrogenrezeptor β), welcher von einer cDNA kodiert wird, wobei gegebenenfalls eine oder mehrere cDNAs,
   die für SLIM3, AR oder ERβ kodieren,
      modifiziert sind und dabei mindestens 85% homolog zu der cDNA Sequenz sind,
   die für das natürliche SLIM3, AR oder ERβ kodiert,
      ohne daß durch die Modifizierung der cDNAs die Funktion des jeweiligen, exprimierten Proteins im Vergleich zu dem natürlichen Protein beeinflußt wird.

Bevorzugt ist die erfindungsgemäße Verwendung von SLIM3, wobei die Kern-Rezeptoren AR und ERβ die Transkription von Zellen kontrollieren.

Mehr bevorzugt ist die erfindungsgemäße Verwendung von SLIM3 als funktioneller Koaktivator für die Kern - Rezeptoren AR und ERβ.

Am meisten bevorzugt ist die erfindungsgemäße Verwendung von SLIM3 zur Steigerung der Transkription von den Kern - Rezeptoren AR und ERβ.

### Definitionen

### Modifizierung von SLIM3 und Kern - Rezeptoren AR und ERβ mit allelischen und posttranslationalen Modifikationen:

### Allelische Modifikationen

Die meisten Deletionen, Insertionen und Substitutionen scheinen keine durchgreifende Änderung in der Charakteristik der Proteine SLIM3, AR und ERβ zur Folge zu haben. Da es schwer ist, den genauen Effekt einer Substitution, einer Deletion oder einer Insertion im voraus anzugeben, muß die Funktion des veränderten Proteins mit der Funktion des bekannten Proteins verglichen werden. Als Standard für SLIM3 dient das Protein gemäß der Publikation von K.K. CHAN et al. (1998) Moelcular cloning and characterization of FHL2, a novel LIM domain protein preferentially expressed in human heart, Gene, Vol. 210, pp 345 - 350 und M. GENINI et al. (1997) Substractive cloning and characterization of DRAL, a novel LIM - domain protein down - regulated in rhabdomyosarcoma, DNA Cell Biol, Vol. 16, pp 433 - 442 und M.J. MORGAN and A.J.A. MADGWICK (1996) Biochem. Biophys, Res. Commun., Vol. 225, pp 632 - 638 (= natürliche SLIM3). Für die Kern - Rezeptoren (= natürliche AR und ERβ) dienen als Standard die Proteine (= natürliche AR und ERβ), welche in den Publikationen über AR und ER beschrieben sind: für AR: A.C.B. CATO et al. (1998) The androgen receptor as mediator of gene expression and signal transduction pathways, TEM, Vol. 9, pp 150 - 154 und Z.X. ZHOU, et al. (1994) The androgen receptor: an overview; Recent Prog Horm Res, Vol. 49, pp 249-274 und E.M. WILSON et al. (1991) Molecular analysis of the androgen receptor; Ann N Y Sci, Vol. 637, pp 56 - 63; weiterhin für ERβ: V. GIGUERE et al. (1998) Estrogen receptor beta: re-evaluation of estrogen and antiestrogen signaling; Steriods Vol. 63, pp 335 - 339 und G.G. KUIPER et al. (1997) The novel estrogen receptor subtype: potential role in the cell- and promoter-specific actions of estrogens and anti - estrogens, FEBS Lett, Vol. 410: pp 87-90.

Mutationen werden durch die Homologie (Similarity) zweier zum Vergleich anstehender Proteine oder Gene definiert.

Aminosäuren können wie in der Tabelle 1 dargestellt substituiert werden, ohne dabei die Funktion des jeweiligen Proteins wesentlich zu beeinflussen. In jedem einzelnen Fall ist durch den Aktivitätstest zu entscheiden, welchen Einfluß die Veränderung auf die Funktion des Proteins hat.
Die Funktionen oder die immunologische Identität werden wesentlich verändert, wenn Substituenten gewählt werden, die bei der Substituierung weniger konservativ als die in Tabelle 1 gezeigten Aminosäuren sind. Derartige wesentlichen Veränderungen lassen sich durch Substituierungen mit Aminosäuren erzielen, die sich mehr in ihrer Struktur und in den funktionellen Gruppen unterscheiden. Wesentliche Veränderungen wirken sich dahingehend aus, daß die dreidimensionale Struktur verändert wird und/oder daß zum Beispiel die Faltblatt-Struktur oder die helikale Struktur beeinflußt wird. Auch Wechselwirkungen der Ladungen und der hydrophoben Ketten sind bei den Veränderungen zu beachten.

**Tabelle 1**

| **Übliche Substituierung von Aminosäuren in einem Protein** | |
|---|---|
| Ursprüngliche Aminosäure | Beispielsweise vorgenommene Substituierung |
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Derartige Analysen über Substituierungen lassen sich leicht bewerkstelligen. Dabei wird je eine Aminosäure in einer Position gegen bevorzugt Alanin oder eine weitere Aminosäure ausgetauscht. Nach der Synthese des modifizierten Proteins wird die Funktion des veränderten Proteins gemessen, indem bei SLIM3 die Bindungsfähigkeit und die Fähigkeit, ein Kofaktor zu sein, getestet wird. Bei den Rezeptoren sind andere Tests sinnvoll, welche die Funktion, SLIM3 zu binden, und welche die Funktion, die Transkription zu beeinflussen, messen. Die Funktionen sind in den jeweiligen Literaturstellen zu AR und ERß beschrieben.

### Homologie auf Ebene der Aminosäuresequenzen:

Der Ausdruck Homologie umfaßt ähnliche Aminosäuren (zum Beispiel wie in Tabelle 1 ausgedrückt) und Lücken in den Sequenzen der Aminosäuren (Homologie = similarity). Die Proteine in der Anmeldung haben Aminosäure-Sequenzen, die eine Homologie von mindestens 90 %, bevorzugt 95 %, mehr bevorzugt 98 % und am meisten bevorzugt 99 % der beschriebenen Strukturen (SLIM3 und die zwei Kern - Rezeptoren) besitzt.

### Homologie auf der DNA - Ebene:

Homologie bei Nukleotidsequenzen bedeutet, daß zwei Polynukleotide oder entsprechende Sequenzen davon, wenn sie optimal aneinander gelegt und ineinandergreifen, identisch sind. Die Homologie kann jedoch auch nur teilweise vorliegen. So soll bei SLIM3 und den Rezeptoren eine Homologie von mindestens 85%, bevorzugt 92% mehr bevorzugt 98% und am meisten bevorzugt 99% vorliegen. Die Homologie wird über die Hybridisierung zweier Nukleotidstränge ermittelt, wie dieses in J. COOMBS (1994) Dictionary of Biotechnology, Stockton Press, New York beschrieben ist. Die Homologie kann auch so gemessen werden, wie es in R. KNIPPERS, Molekulare Genetik, 1982, dritte Auflage, Georg Thieme Verlag Stuttgart, New York beschrieben ist.

### Posttranslationale Modifikationen

Unter den zuvor erwähnten posttranslationalen Modifikationen versteht man Veränderungen, die während oder nach der Translation auftreten. Hierzu zählen die Glykosylierung, die Ausbildung von Disulfid - Brücken, die chemische Modifikationen der Aminosäuren, so zum Beispiel die Sulfatierung, die im Zusammenhang mit dem Hirudin beschrieben ist. (J.W. FENTON (1989) "Thrombin Interactions with Hirudin", Seminars in Thrombosis and Hemostasis **15**: 265-268). Weiterhin fallen unter diese Modifikationen auch die Phosphorylierung und die Proteolyse.

Die Glykosylierung ist eine wesentliche Funktion des endoplasmatischen Retikulums und/oder des Golgi-Apparates. Die Sequenz und die Verästelung der Oligosaccharide wird in dem endoplasmatischem Retikulum gebildet und in dem Golgi-Apparat verändert. Die Oligosaccharide können N - verknüpfte Oligosaccharide (Asparagin - verknüpfte) oder O - verknüpfte Oligosaccharide (Serin -, Threonin - oder Hydroxylysin-verknüpfte) sein. Die Form der Glykosylierung ist von dem produzierenden Zelltyp und von der Art abhängig, von der der entsprechende Zelltyp stammt. Das Ausmaß und die Art der Glykosylierung kann durch Substanzen beeinflußt werden, wie es in der europäischen Publikation EP 0 222 313 beschrieben ist. Die Variierung der Glykosylierung kann die Funktion des Proteins verändern.
Proteine bilden häufig kovalente Bindungen innerhalb der Ketten aus. Diese Disulfid - Brücken werden zwischen zwei Cysteinen hergestellt. Dabei wird das Protein spezifisch gefaltet. Die Disulfid - Brücken stabilisieren die dreidimensionale Struktur der Proteine.
SLIM3 - Modifizierungen werden dadurch bestimmt, daß die Funktion gemäß der Beispiele von dem in der Literatur beschriebenen SLIM3 mit der Modifizierung verglichen wird. Ist die Funktion im wesentlichen ähnlich, so liegt eine Modifizierung nach dem Anspruch vor.
Kern - Rezeptor AR - Modifizierungen werden dadurch bestimmt, daß die Funktionen der DNA-Bindung, Konformation, Kofaktor - Bindung und die transkriptionelle Eigenschaften von dem in der Literatur beschriebenen Kern-Rezeptor AR mit dem der Modifizierungen verglichen werden (A.C.B. CATO et al. (1998) The androgen receptor as mediator of gene expression and signal transduction pathways, TEM, Vol. 9, pp 150 - 154 und Z.X. ZHOU, et al. (1994) The androgen receptor: an overview. Recent Prog Horm Res, Vol. 49, pp 249 - 274 und E.M. WILSON et al. (1991) Molecular analysis of the androgen receptor. Ann N Y Sci, Vol. 637, pp 56 - 63 und J. TORCHIA et al. (1998) Co - activators and co - repressors in the integration of transcriptional responses. Curr Opin Cell Biol. Vol. 10, pp 373 - 383 und D.J. MANGELSDORF et al. (1995) The nuclear receptor superfamily: the second decade, Cell, Vol. 83, pp 835 - 839). Sind die Funktionen im wesentlichen ähnlich, so liegt eine Modifizierungen nach dem Hauptanspruch vor.

Kern - Rezeptor ERβ - Modifizierungen werden dadurch bestimmt, daß die Funktionen der DNA-Bindung, Konformation, Kofaktor - Bindung und die transkriptionelle Eigenschaften von dem in der Literatur beschriebenen kern-Rezeptor ERβ mit dem der Modifizierungen verglichen werden (V. GIGUERE et al. (1998) Estrogen receptor beta: re-evaluation of estrogen and antiestrogen signaling. Steriods Vol. 63, pp 335 - 339 und G.G. KUIPER et al. (1997) The novel estrogen receptor subtype: potential role in the cell- and promoter - specific actions of estrogens and anti - estrogens, FEBS Lett, Vol. 410: pp 87 - 90 und J. TORCHIA et al. (1998) Co - activators and co-repressors in the integration of transcriptional responses. Curr Opin Cell Biol.

Vol. 10, pp 373 - 383 und D.J. MANGELSDORF et al. (1995) The nuclear receptor superfamily: the second decade, Cell, Vol. 83, pp 835 - 839). Sind die Funktionen im wesentlichen ähnlich, so liegt eine Modifizierungen nach dem Hauptanspruch vor.
Ligand: Zu den Liganden zählen alle natürlichen und synthetisch hergestellten Substanzen, die an einen Rezeptor und / oder an SLIM3 binden können. Der Ligand kann auch ein gegen einen Rezeptor und / oder SLIM3 gerichteter Antikörper sein. Liganden können Agonisten oder Antagonisten sein.

### Screening Verfahren

Die Erfindung umfaßt weiterhin ein in vitro Verfahren zum Identifizieren (Screening) von Liganden,
welche die Interaktion von SLIM3 oder Modifizierungen davon und von mindestens einem der kern - Rezeptoren AR oder ERβ oder Modifizierungen davon so beeinflussen,
   daß die Transkription,
die von dem Kern - Rezeptor kontrolliert wird,
   gesteigert oder vermindert wird,
wobei von SLIM3 (= Protein) oder einer Modifizierung davon
   zur Bindung an mindestens einen der kern - Rezeptoren
   (i) AR (Androgenrezeptor) (= Protein) oder einer Modifizierung davon und
   (ii) ERβ (Östrogenrezeptor β) (= Protein) oder einer Modifizierung davon,
gemäß der Erfindung verwendet wird.

Die Modifizierungen sind gemäß der vorherigen Beschreibung definiert. Bevorzugt ist ein erfindungsgemäßes Verfahren, wobei der Ligand ein Antagonist oder Agonist ist. Hierdurch lassen sich gezielt Antagonisten und Agonisten finden, die auf einzelne Organe wirken, da SLIM3 spezifisch in einigen Geweben exprimiert wird. Die gefundenen Liganden können somit als Medikament oder als Leitstruktur zum Auffinden eines Medikaments eingesetzt werden.
Vergleichbare Screening Methoden sind beschrieben (A.C.B. CATO et al. (1998) The androgen receptor as mediator of gene expression and signal transduction pathways, TEM, Vol. 9, pp 150 - 154 und G. Zhou et al. (1998) Nuclear receptors have distinct affinities for coactivators: Characterization by fluorescence resonance energy transfer, Mol. Endocrinol. Vol. 12, pp 1594-1604).
Indikatoren für Transkription sind beschrieben in J. Sambrook et al. (1989) Molecular cloning. Cold Spring Harbour Laboratory Press, New York.

Ebenfalls zeigten weitere GST - Protein - Interaktionsversuche, daß SLIM3 mit bekannten Koaktivatoren TIF2, RIP140 und AIB/ACTR interagiert. Jedoch besteht keine Interaktion zwischen SLIM3 und dem Koaktivator SRC1 *in vitro.*

### Beispiele

### Expression von SLIM3 in verschiedenen Geweben:

Northern Blots und *in situ* Analysen haben gezeigt, daß SLIM3 während der Embryonalentwicklung vom Tag neun der Entwicklung exprimiert wird. Vom zwölften Tag der Embroynalentwicklung wird SLIM3 weiterhin in der viszeralen und cardiovaskularen glatten Muskulatur nachgewiesen. Diese spezifische Expression dauert bis ins Alter an. Keine Expression war in den folgenden Geweben feststellbar: Gehirn, Lunge, Leber, Nieren, Bauchspeicheldrüse, Milz, Thymus und den periphären Blut - Leukozyten. Die Versuche wurden gemäß M. MOSER et al. (1997) Comparative analysis of AP-2 alpha and AP-2 beta gene expression during mice embryogenesis. Dev Dyn, Vol. 208, pp 115- 124 ausgeführt. Zellkulturen und transiente Transfektionsversuche:
293, CV1 und A10 Zellen wurden in Dulbecco's modified Eagle's medium (DMEM) kultiviert, in dem sich 10% oder 20% (A10) fötales Kälberserum befand. HL-1 wurden in Ex-cell 320 kultiviert, wie dieses beschrieben ist in W.C. CLAYCOMB et al. (1998) HL-1 cells: a cardiac muscle cell line that contracts and retains phenotypic characteristics of the adult cardiomyocyte, Proc Natl Acad Sci USA, Vol. 95, pp 2979 - 2984. Transiente Transfektionsversuche in 293 Zellen wurde nach dem Standard der Kalziumphosphat Koprezipitationstechnik ausgeführt (Greiner, E. F., J. Kirfel, H. Greschik, U. Dörflinger, P. Becker, A. Mercep, and R. Schüle. 1996. Functional analysis of retinoid Z receptor β, a brain-specific nuclear orphan receptor. Proc. Natl. Acad. Sci. USA 93: 10105-10110).
A10 und CV-1 wurden transfiziert, indem DOTAP entsprechen der Herstellungsempfehlung (manufacture's recommendation) von Boehringer Mannheim eingesetzt wurde. HL-1 wurden transfiziert, indem Effecten entsprechen der Herstellungsempfehlung (manufacture's recommendation) von Qiagen eingesetzt wurde. Die Zellen wurden nach 22 Stunden mit oder ohne Hormonen inkubiert und die Luziferase - Aktivität wurde entsprechend der Empfehlung von Promega in a Luminometer ML3000 (Dynatech) gemessen. Die relativen Lichteinheiten wurden gegen die Proteinkonzentration entsprechend dem Bradford Farbtest (BioRad) abgeglichen. Alle Experimente wurden fünfmal wiederholt.

### SLIM3 spezifische Interaktion mit dem Ligand - aktiviertem Androgenrezeptor :

Um die Interaktion zwischen SLIM3 und dem Androgenrezeptor zu messen, wurde der Flüssig β-Galactosidase Versuch verwendet. Daher wurde die DNA des Androgenrezeptors durch eine GAL4 DNA Bindungsdomäne ersetzt. Das erhaltene chimäre Protein (AGA), das den humanen Androgenrezeptor-N-Terminus und die Liganden bindende Androgenrezeptor - Domäne (LBD) enthält, wurde als Zielprotein verwendet. Slim3, das mit der GAL4 Aktivator-Domäne (Slim3-AD) fusioniert war, wurde als bindendes Protein eingesetzt. Slim3-AD, das mit AGA in einer Liganden - abhängigen Art verbunden ist, erhöht die β-Galactosidase Reporter Gen Aktivität in Hefe um mehr als das zehnfache.

### SLIM3 interagiert spezifisch mit einigen Kernrezeptoren :

Um die Interaktion zwischen SLIM3 und den Kernrezeptoren zu messen, wurde das GST-Slim3 Fusionsprotein mittels Glutation - Sepharose immobilisiert und mit einigen *in vitro* translatierten ³⁵S - Methionin markierten Kernrezeptoren inkubiert. Der vollständige humane Androgenrezeptor bindet an GST-Slim3. Diese Interaktion ist unter Standardbedingungen nicht ligandabhängig. Der vollständige humane Androgenrezeptor bindet spezifisch an GST-Slim3, jedoch nicht an das GST Protein alleine. Um die Spezifität zwischen dem humanen Androgenrezeptor und SLIM3 abzusichern, wurde ein weiterer GST - Protein - Interaktionsversuch ausgeführt (GST - Protein-Interaktionsassay). Dieser Versuch ist beschrieben in E. PFITZNER et al. (1995) Functional antagonism between the retinoid acid receptor and the viral transactivator BZLF1 ist mediated by protein - protein interactions. Proc Natl Acad Sci USA Vol. 92, pp 12265- 12269. Spezifische liganden - unabhängige Interaktionen zwischen dem GST - SLIM3 - Fusionsprotein und dem ERβ Rezeptor konnten beobachtet werden. Alle anderen getesteten kernrezeptoren, zum Beispiel Progesteronrezeptor alpha und beta, Glucocoritcodidrezeptor und Estrogenrezeptor alpha zeigten keine Bindung an SLIM3.
Ebenfalls zeigten weitere GST - Protein - Interaktionsversuche, daß SLIM3 mit bekannten Koaktivatoren TIF2, RIP140 und AIB/ACTR interagiert. Jedoch besteht keine Interaktion zwischen SLIM3 und dem Koaktivator SRC1 *in vitro.* SLIM 3 ist ein funktioneller Koaktivator:
In Experimenten konnte gezeigt werden, daß SLIM3 nicht an DNA bindet. SLIM3 ist ein Koaktivator für bestimmte Rezeptoren und in bestimmten Zelltypen. Gal-SLIM3, welches sich in ein Expressionsplasmid befand, wurde in verschiedenen Säuger - Zell - Linien kotransfiziert. Dabei stellte sich die Frage, ob Gal-SLIM3 fähig ist, ein Luziferase - Reporter - Gen zu transaktivieren, das durch Gal - Bindungsstellen kontrolliert wird, wie zum Beispiel die drei Gal4-Bindungsstellen aufwärts (upstream) vom Thymidinkinase - Promotor [(GAL)3-TKLuC]. Gal-SLIM3 konnte eine Reportergen - Expression in der Herzmuskel-Zell - Linie HL1, in der glatten Muskel - Zell - Linie A10 und in der embryonalen Nieren - Zell - Linie 293 transaktivieren.
SLIM3 ist ein funktioneller Androgenrezeptor Koaktivator: Die Reporter-Plasmide MMTV-LUC oder Proteasin - Luc wurde zusammen mit einer konstanten Menge des Androgenrezeptor - Expression - Plasmids in 293 Zellen kotransfiziert. Wurde der synthetische Androgenrezeptoragonist R1881 hinzugegeben, so wurde das Reportergen abhängig von den Liganden und Rezeptoren transaktiviert. Eine Kotransfektion von einer steigenden Menge an SLIM3 - Expressions - Plasmiden führte zu einer weiteren dreifachen bis fünffachen Superinduktion des Reportergens.
Ähnliche Resultate wurde in CV1 Zellen erhalten. Diese Daten zeigen, daß SLIM3 ein Koaktivator für den Kernrezeptor AR ist.

Im Vergleich mit anderen bekannten Koaktivatoren besitzt SLIM3 eine außergewöhnliche Spezifität. SLIM3 reguliert lediglich die Aktivität von zwei Kernrezeptoren, nämlich AR und ERβ. Kein anderer Kofaktor ist bekannt, der eine solche Funktion erfüllt. Somit kann SLIM3 mit den Kernrezeptoren AR und ERβ die entsprechende Translation in bestimmten Geweben oder Zellen aktivieren.

## Patentansprüche

1. In vitro Verwendung
von SLIM3 (= Protein) oder einer Modifizierung davon
zur Bindung an mindestens einen der Kern - Rezeptoren
(i) AR (Androgenrezeptor) (= Protein) oder einer Modifizierung davon und
(ii) ERβ (Östrogenrezeptor β) (= Protein) oder einer Modifizierung davon,
wobei die Modifizierung darin besteht, daß bis zu zehn Aminosäuren in dem jeweiligen modifizierten Protein gegenüber dem entsprechenden natürlichen Protein deletiert, substituiert oder inseriert sind,
ohne dabei die Funktion des jeweiligen modifizierten Proteins im Vergleich zu dem entsprechenden natürlichen Protein zu beeinflussen.

2. In vitro Verwendung
der Aminosäuresequenz für SLIM3 (= Protein),
welches von einer cDNA kodiert wird,
zur Bindung an mindestens eine Aminosäuresequenz für die Kern - Rezeptoren (= Protein)
(i) AR (Androgenrezeptor), welcher von einer cDNA kodiert wird, und
(ii) ERβ (Östrogenrezeptor β), welcher von einer cDNA kodiert wird,
wobei gegebenenfalls eine oder mehrere cDNAs,
die für SLIM3, AR oder ERβ kodieren,
modifiziert sind und dabei mindestens 85% homolog zu der cDNA Sequenz sind,
die für das natürliche SLIM3, AR oder ERβ kodiert,
ohne daß durch die Modifizierung der cDNAs die Funktion des jeweiligen, exprimierten Proteins im Vergleich zu dem natürlichen Protein beeinflußt wird.

3. Verwendung nach einem der Ansprüche 1 oder 2, wobei die kern-Rezeptoren AR und ERβ die Transkription von Zellen kontrollieren.

4. Verwendung nach einem der vorherigen Ansprüche, wobei SLIM3 ein funktioneller Koaktivator für die Kern - Rezeptoren AR und ERβ ist.

5. Verwendung von SLIM3 nach einem der vorherigen Ansprüche zur Steigerung der Transkription von den Kern - Rezeptoren AR und ERβ.

6. In vitro Verfahren zum Identifizieren (Screening) von Liganden,
welche die Interaktion von SLIM3 oder Modifizierungen davon und von mindestens einem der Kern - Rezeptoren AR oder ERβ oder Modifizierungen davon so beeinflussen,
daß die Transkription,
die von dem Kern - Rezeptor kontrolliert wird,
gesteigert oder vermindert wird,
wobei von SLIM3 oder einer Modifizierung davon
zur Bindung an mindestens einen der Kern - Rezeptoren
(i) AR oder einer Modifizierung davon und
(ii) ERβ oder einer Modifizierung davon,
gemäß einem der vorherigen Ansprüche verwendet wird.

7. Verfahren nach Anspruch 6, wobei der Ligand ein Antagonist oder Agonist ist.

## Claims

1. Use of SLIM3 (= protein) or a modification thereof in vitro for binding to at least one of the nuclear receptors
(i) AR (androgen receptor) (= protein) or a modification thereof, and
(ii) ERβ (oestrogen receptor β) (= protein) or a modification thereof,
with the modification being that up to ten amino acids in the particular modified protein have been deleted, substituted or inserted as compared with the corresponding natural protein,
without this thereby affecting the function of the particular modified protein as compared with the corresponding natural protein.

2. Use of the amino acid sequence for SLIM3 (= protein), which is encoded by a cDNA, in vitro for binding to at least one amino acid sequence for the nuclear receptors (= protein)
(i) AR (androgen receptor), which is encoded by a cDNA, and
(ii) ERβ (oestrogen receptor β), which is encoded by a cDNA,
with, where appropriate; one or more cDNAs, which encode SLIM3, AR or ERβ,
being modified and, in this connection, being at least 85% homologous to the cDNA sequence,
which encodes the natural SLIM3, AR or ERβ, without the modification of the cDNAs affecting the function of the particular, expressed protein as compared with the natural protein.

3. Use according to Claim 1 or 2, where the nuclear receptors AR and ERβ control the transcription of cells.

4. Use according to one of the preceding claims, where SLIM3 is a functional coactivator for the nuclear receptors AR and ERβ.

5. Use of SLIM3 according to one of the preceding claims for increasing the transcription of the nuclear receptors AR and ERβ.

6. In-vitro process for identifying (screening) ligands
which influence the interaction of SLIM3, or modifications thereof, and at least one of the nuclear receptors AR or ERβ, or modifications thereof,
such that the transcription
which is controlled by the nuclear receptor is increased or diminished,
with SLIM3, or a modification thereof, being used, according to the one of the preceding claims, for binding to at least one of the nuclear receptors
(i) AR or a modification thereof, and
(ii) ERβ or a modification thereof.

7. Process according to Claim 6, where the ligand is an antagonist or agonist.

## Revendications

1. Utilisation in vitro de SLIM3 (= protéine) ou d'une modification de celle-ci pour la liaison à au moins un des récepteurs nucléaires
(i) AR (récepteur des androgènes) (= protéine) ou une modification de celui-ci et
(ii) ERβ (récepteur des oestrogènes β) (= protéine) ou une modification de celui-ci, la modification résidant en ce que jusqu'à dix acides aminés dans chaque protéine modifiée sont supprimés, substitués ou insérés par rapport à la protéine naturelle correspondante, sans influencer en l'occurrence la fonction de chaque protéine modifiée par rapport à la protéine naturelle correspondante.

2. Utilisation in vitro de la séquence d'acides aminés pour la SLIM3 (= protéine) qui est codée par un cADN pour la liaison à au moins une séquence d'acides aminés pour les récepteurs nucléaires (= protéine)
(i) AR (récepteur des androgènes) qui est codé par un cADN et
(ii) ERβ (récepteur des oestrogènes β) qui est codé par un cADN, un ou plusieurs cADN qui codent pour la SLIM3, l'AR ou l'ERβ, étant le cas échéant modifiés et en l'occurrence homologues par rapport à au moins 85% à la séquence cADN qui code pour la SLIM3 naturelle, l'AR naturel ou l'ERβ naturel, sans que la fonction de chaque protéine exprimée ne soit influencée par rapport à la protéine naturelle par la modification des cADN.

3. Utilisation selon l'une quelconque des revendications 1 ou 2, les récepteurs nucléaires AR et ERβ contrôlant la transcription de cellules.

4. Utilisation selon l'une quelconque des revendications précédentes, la SLIM3 étant un coactivateur fonctionnel pour les récepteurs nucléaires AR et ERβ.

5. Utilisation de SLIM3 selon l'une quelconque des revendications précédentes pour augmenter la transcription des récepteurs nucléaires AR et ERβ.

6. Procédé in vitro pour l'identification (screening) de ligands, qui influencent l'interaction de la SLIM3 ou de modifications de celle-ci et d'au moins un des récepteurs nucléaires AR ou ERβ ou des modifications de ceux-ci de telle manière que la transcription qui est contrôlée par le récepteur nucléaire soit augmentée ou diminuée, la SLIM3 ou une modification de celle-ci étant utilisée pour la liaison à au moins un des récepteurs nucléaires
(i) AR ou une modification de celui-ci et
(ii) ERβ ou une modification de celui-ci,
selon l'une quelconque des revendications précédentes.

7. Procédé selon la revendication 6, dans lequel le ligand est un antagoniste ou un agoniste.
